# EUROPEAN PATENT SPECIFICATION

(11) **EP 3 327 520 B1**
(45) Date of publication and mention of the grant of the patent: **17.06.2020**
(21) Application number: 15899011.9
(22) Date of filing: 17.11.2015
(51) Int. Cl.: H04M 1/725, A61B 5/053, G06F 1/16, A61B 5/00

(54) **WATCH-TYPE TERMINAL**
UHRENARTIGES ENDGERÄT
TERMINAL DE TYPE MONTRE

(30) Priority: 20.07.2015 US 201562194324 P; 22.09.2015 KR 20150134152
(43) Date of publication of application: 30.05.2018
(73) Proprietor: LG Electronics Inc., Yeongdeungpo-gu Seoul 07336 (KR)
(72) Inventor: SHIM, Hongjo, Seoul 06772 (KR); WOO, Sungho, Seoul 06772 (KR); LEE, Hyunok, Seoul 06772 (KR); SOHN, Youngho, Seoul 06772 (KR); KIM, Seonghyok, Seoul 06772 (KR); PARK, Mihyun, Seoul 06772 (KR); PARK, Jisoo, Seoul 06772 (KR)
(74) Representative: Schornack, Oliver
(86) International application number: PCT/KR2015/012320
(87) International publication number: WO 2017/014364

(56) References cited:
- EP-A1- 3 078 325
- JP-A- H11 202 065
- JP-A- 2001 145 607
- JP-A- 2002 355 230
- JP-A- 2009 519 739
- KR-A- 20150 079 440
- KR-B1- 100 330 746
- US-A1- 2010 076 331

## Description

### [Technical Field]

The present invention relates to a watch-type terminal that may be worn on the wrist of a user

### [Background Art]

Terminals may generally be classified as glass type terminals (mobile (portable) terminals) and stationary terminals according to a moveable state. The glass type terminals may be also classified as handheld terminals and vehicle mount terminals according to a user's carriage method.

As functions of terminals become more diversified, the terminals can support more complicated functions such as capturing images or video, reproducing music or video files, playing games, receiving broadcast signals, and the like. By comprehensively and collectively implementing such functions, the terminals may be embodied in the form of multimedia players or devices. In order to support and increase functions of terminals, improvement of structural parts and/or software parts of terminals may be taken into consideration.

Recently, various functions for collecting biometric information by a sensor, or the like, included in a wearable-type terminal formed to be worn on a part of a human body have been studied. Body fat measurement, in which a measurement sensor is required to be mounted in different areas of the user's body, has unique characteristics that the user should contact the body of two different areas, and installation of the sensor increases a weight of a terminal.

EP 3 078 325 A1 is a European patent application falling within the terms of Art. 54(3) EPC and refers to an apparatus for obtaining biological information, the apparatus including a main body and a plurality of straps connected to the main body and wearable on a wrist of a user. When the user wears the apparatus for obtaining bio information, a first current electrode and a first voltage electrode contact the user's wrist. The device further includes a second current electrode and a second voltage electrode arranged to be exposed to the outside of the apparatus.

JP 2001-145607 refers to a portable body fat measuring instrument configured as a wristwatch. The instrument includes a pair of electrodes on a front side and a pair of electrodes on a back side.

KR 2015-0079440 refers to a wearable device which includes a function part comprising a socket for connection with a band part for fixating the function unit on a user's wrist.

### [Disclosure]

### [Technical Problem]

An aspect of the present disclosure improves wearability of a watch-type terminal capable of collecting body fat measurement information while a user is wearing the watch-type terminal.

### [Technical Solution]

According to an aspect of the present disclosure, a watch-type terminal according to the present invention is defined in claim 1. Preferred embodiments are defined by the dependent claims. The embodiments which do not fall within the scope of the claims do not describe part of the present invention. A watch-type terminal may include: a main body; a band part connected to the main body and formed to be wearable on the wrist; an electrode unit disposed in a region of each of the main body and the band so as to come into contact with the body of a user, and generating a current; and a controller calculating an impedance value on the basis of a voltage sensed by the electrode unit, wherein the electrode unit includes a first electrode member which comes into contact with the wrist when the watch-type terminal is worn around the wrist and a second electrode member exposed to the outside.

In an example related to the present invention, a second band including the first and second electrode members are formed to be separable from the main body, and thus, the user may selectively connect the second band to the main body only when the user wants to be provided with a body fat measurement result.

In an example related to the present invention, the first electrode member is formed in a fastener connecting first and second bands, the electrode unit may be formed without any additional structure, simplifying the structure of the watch-type terminal and reducing a weight thereof.

### [Advantageous Effects]

According to the present invention, when the watch-type terminal is worn on the user's wrist, a Tx electrode or a Rx electrode is kept in contact with the user's wrist, and thus, the user may bring a part of his body into contact with the other electrode to receive a body fat measurement result more rapidly. Since the first and second electrode members according to the present invention form components of the watch-type terminal, an additionally connected measurement sensor is not necessary.

In addition, since a band in which an electrode unit is disposed is separable, the band may be replaced with another band not including the electrode unit. That is, a body fat measurement result may be received only when the user wants it, and biometric information required for the user may be collected by a band in which another sensor measuring a biometric signal is installed.

### [Description of Drawings]

FIG. 1 is a block diagram illustrating a mobile terminal related to the present invention.
FIG. 2 is a perspective view of a mobile terminal related to the present invention.
FIGS. 3A to 3C are conceptual views illustrating a structure in which first and second bands are fixed to main body.
FIGS. 4A to 5B are conceptual view illustrating a body fat measurement principle.
FIG. 6A is a conceptual view illustrating a structure of a watch-type terminal according to another embodiment.
FIG. 6B is a partially enlarged view illustrating a connection part.
FIG. 6C is a cross-sectional view of a connection part.
FIGS. 7A to 7D are conceptual views illustrating a watch-type terminal including an electrode unit according to various embodiments.

### [Best Modes]

Description will now be given in detail according to exemplary embodiments disclosed herein, with reference to the accompanying drawings. For the sake of brief description with reference to the drawings, the same or equivalent components may be provided with the same or similar reference numbers, and description thereof will not be repeated. In general, a suffix such as "module" and "unit" may be used to refer to elements or components. Use of such a suffix herein is merely intended to facilitate description of the specification, and the suffix itself is not intended to give any special meaning or function. In the present disclosure, that which is well-known to one of ordinary skill in the relevant art has generally been omitted for the sake of brevity. The accompanying drawings are used to help easily understand various technical features and it should be understood that the examples and embodiments presented herein are not limited by the accompanying drawings.

Mobile terminals presented herein may be implemented using a variety of different types of terminals. Examples of such terminals include cellular phones, smart phones, user equipment, laptop computers, digital broadcast terminals, personal digital assistants (PDAs), portable multimedia players (PMPs), navigators, portable computers (PCs), slate PCs, tablet PCs, ultra books, wearable devices (for example, smart watches, smart glasses, head mounted displays (HMDs)), and the like.

By way of non-limiting example only, further description will be made with reference to particular types of mobile terminals. However, such teachings apply equally to other types of terminals, such as those types noted above. In addition, these teachings may also be applied to stationary terminals such as digital TV, desktop computers, and the like.

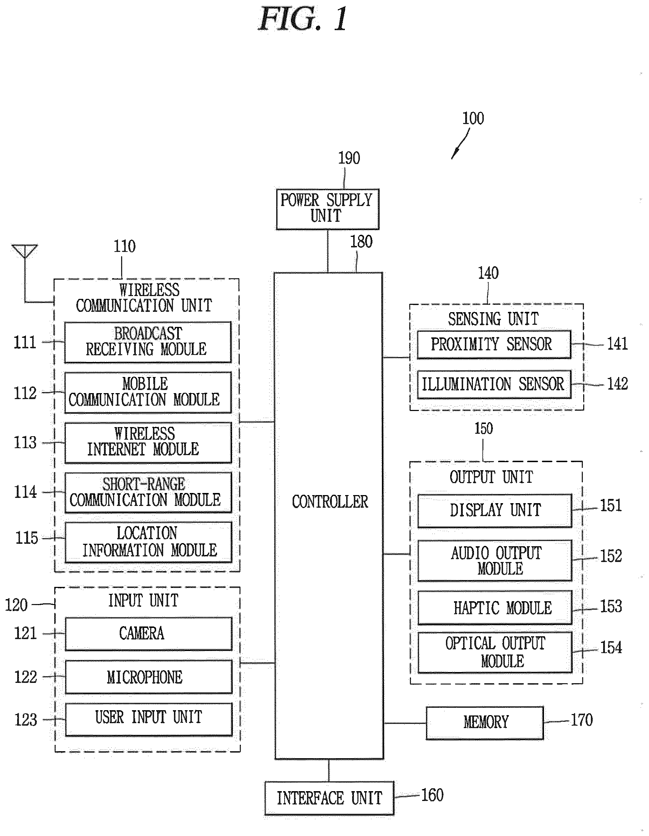

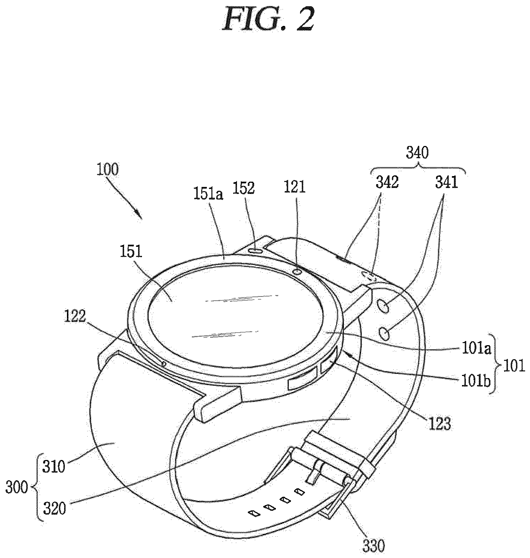

FIG. 1 is a block diagram illustrating a mobile terminal related to the present invention, and FIG. 2 is a perspective view of a mobile terminal related to the present invention.

The watch-type terminal 100 is shown having components such as a wireless communication unit 110, an input unit 120, a sensing unit 140, an output unit 150, an interface unit 160, a memory 170, a control unit 180, and a power supply unit 190. It is understood that implementing all of the illustrated components is not a requirement, and that greater or fewer components may alternatively be implemented.

The wireless communication unit 110 typically includes one or more modules which permit communications such as wireless communications between the watch-type terminal 100 and a wireless communication system, communications between the watch-type terminal 100 and another mobile terminal, communications between the watch-type terminal 100 and an external server.

Further, the wireless communication unit 110 typically includes one or more modules which connect the watch-type terminal 100 to one or more networks. To facilitate such communications, the wireless communication unit 110 includes one or more of a broadcast receiving module 111, a mobile communication module 112, a wireless Internet module 113, a short-range communication module 114, and a location information module 115.

The input unit 120 includes a camera 121 for obtaining images or video, a microphone 122, which is one type of audio input device for inputting an audio signal, and a user input unit 123 (for example, a touch key, a push key, a mechanical key, a soft key, and the like) for allowing a user to input information. Data (for example, audio, video, image, and the like) is obtained by the input unit 120 and may be analyzed and processed by control unit 180 according to device parameters, user commands, and combinations thereof.

The sensing unit 140 is typically implemented using one or more sensors configured to sense internal information of the mobile terminal, the surrounding environment of the mobile terminal, user information, and the like. For example, in FIG. 1A, the sensing unit 140 is shown having a proximity sensor 141 and an illumination sensor 142. If desired, the sensing unit 140 may alternatively or additionally include other types of sensors or devices, such as a touch sensor, an acceleration sensor, a magnetic sensor, a G-sensor, a gyroscope sensor, a motion sensor, an RGB sensor, an infrared (IR) sensor, a finger scan sensor, a ultrasonic sensor, an optical sensor (for example, camera 121), a microphone 122, a battery gauge, an environment sensor (for example, a barometer, a hygrometer, a thermometer, a radiation detection sensor, a thermal sensor, and a gas sensor, among others), and a chemical sensor (for example, an electronic nose, a health care sensor, a biometric sensor, and the like), to name a few. The watch-type terminal 100 may be configured to utilize information obtained from sensing unit 140, and in particular, information obtained from one or more sensors of the sensing unit 140, and combinations thereof.

The output unit 150 is typically configured to output various types of information, such as audio, video, tactile output, and the like. The output unit 150 is shown having a display unit 151, an audio output module 152, a haptic module 153, and an optical output module 154.

The display unit 151 may have an inter-layered structure or an integrated structure with a touch sensor in order to facilitate a touch screen. The touch screen may provide an output interface between the watch-type terminal 100 and a user, as well as function as the user input unit 123 which provides an input interface between the watch-type terminal 100 and the user.

The interface unit 160 serves as an interface with various types of external devices that can be coupled to the watch-type terminal 100. The interface unit 160, for example, may include any of wired or wireless ports, external power supply ports, wired or wireless data ports, memory card ports, ports for connecting a device having an identification module, audio input/output (I/O) ports, video I/O ports, earphone ports, and the like. In some cases, the watch-type terminal 100 may perform assorted control functions associated with a connected external device, in response to the external device being connected to the interface unit 160.

The memory 170 is typically implemented to store data to support various functions or features of the watch-type terminal 100. For instance, the memory 170 may be configured to store application programs executed in the watch-type terminal 100, data or instructions for operations of the watch-type terminal 100, and the like. Some of these application programs may be downloaded from an external server via wireless communication. Other application programs may be installed within the watch-type terminal 100 at time of manufacturing or shipping, which is typically the case for basic functions of the watch-type terminal 100 (for example, receiving a call, placing a call, receiving a message, sending a message, and the like). It is common for application programs to be stored in the memory 170, installed in the watch-type terminal 100, and executed by the control unit 180 to perform an operation (or function) for the watch-type terminal 100.

The control unit 180 typically functions to control overall operation of the watch-type terminal 100, in addition to the operations associated with the application programs. The control unit 180 may provide or process information or functions appropriate for a user by processing signals, data, information and the like, which are input or output by the various components or activating application programs stored in the memory 170.

As one example, the control unit 180 controls some or all of the components illustrated in FIG. 1 according to the execution of an application program that have been stored in the memory 170. In addition, the controller 180 may combine and operate at least two of the components included in the watch-type terminal 100 to drive the application program.

The power supply unit 190 can be configured to receive external power or provide internal power in order to supply appropriate power required for operating elements and components included in the watch-type terminal 100. The power supply unit 190 may include a battery, and the battery may be configured to be embedded in the terminal body, or configured to be detachable from the terminal body.

At least some of the above components may operate in a cooperating manner, so as to implement an operation or a control method of a glass type terminal according to various embodiments to be explained later. The operation or the control method of the glass type terminal may be implemented on the glass type terminal by driving at least one application program stored in the memory 170.

Referring still to FIG. 1, various components depicted in this figure will now be described in more detail.

Regarding the wireless communication unit 110, the broadcast receiving module 111 is typically configured to receive a broadcast signal and/or broadcast associated information from an external broadcast managing entity via a broadcast channel. The broadcast channel may include a satellite channel, a terrestrial channel, or both. In some embodiments, two or more broadcast receiving modules 111 may be utilized to facilitate simultaneously receiving of two or more broadcast channels, or to support switching among broadcast channels.

The mobile communication module 112 can transmit and/or receive wireless signals to and from one or more network entities. Typical examples of a network entity include a base station, an external mobile terminal, a server, and the like. Such network entities form part of a mobile communication network, which is constructed according to technical standards or communication methods for mobile communications (for example, Global System for Mobile Communication (GSM), Code Division Multi Access (CDMA), CDMA2000(Code Division Multi Access 2000), EV-DO(Enhanced Voice-Data Optimized or Enhanced Voice-Data Only), Wideband CDMA (WCDMA), High Speed Downlink Packet access (HSDPA), HSUPA(High Speed Uplink Packet Access), Long Term Evolution (LTE), LTE-A(Long Term Evolution-Advanced), and the like).

Examples of wireless signals transmitted and/or received via the mobile communication module 112 include audio call signals, video (telephony) call signals, or various formats of data to support communication of text and multimedia messages.

The wireless Internet module 113 is configured to facilitate wireless Internet access. This module may be internally or externally coupled to the watch-type terminal 100. The wireless Internet module 113 may transmit and/or receive wireless signals via communication networks according to wireless Internet technologies.

Examples of such wireless Internet access include Wireless LAN (WLAN), Wireless Fidelity (Wi-Fi), Wi-Fi Direct, Digital Living Network Alliance (DLNA), Wireless Broadband (WiBro), Worldwide Interoperability for Microwave Access (WiMAX), High Speed Downlink Packet Access (HSDPA), HSUPA(High Speed Uplink Packet Access), Long Term Evolution (LTE), LTE-A(Long Term Evolution-Advanced), and the like. The wireless Internet module 113 may transmit/receive data according to one or more of such wireless Internet technologies, and other Internet technologies as well.

In some embodiments, when the wireless Internet access is implemented according to, for example, WiBro, HSDPA,HSUPA, GSM, CDMA, WCDMA, LTE, LTE-A and the like, as part of a mobile communication network, the wireless Internet module 113 performs such wireless Internet access. As such, the Internet module 113 may cooperate with, or function as, the mobile communication module 112.

The short-range communication module 114 is configured to facilitate short-range communications. Suitable technologies for implementing such short-range communications include BLUETOOTH™, Radio Frequency IDentification (RFID), Infrared Data Association (IrDA), Ultra-WideBand (UWB), ZigBee, Near Field Communication (NFC), Wireless-Fidelity (Wi-Fi), Wi-Fi Direct, Wireless USB(Wireless Universal Serial Bus), and the like. The short-range communication module 114 in general supports wireless communications between the watch-type terminal 100 and a wireless communication system, communications between the watch-type terminal 100 and another watch-type terminal 100, or communications between the mobile terminal and a network where another watch-type terminal 100 (or an external server) is located, via wireless area networks. One example of the wireless area networks is a wireless personal area networks.

In some embodiments, another mobile terminal (which may be configured similarly to watch-type terminal 100) may be a wearable device, for example, a smart watch, a smart glass or a head mounted display (HMD), which is able to exchange data with the watch-type terminal 100 (or otherwise cooperate with the watch-type terminal 100). The short-range communication module 114 may sense or recognize the wearable device, and permit communication between the wearable device and the watch-type terminal 100. In addition, when the sensed wearable device is a device which is authenticated to communicate with the watch-type terminal 100, the control unit 180, for example, may cause transmission of data processed in the watch-type terminal 100 to the wearable device via the short-range communication module 114. Hence, a user of the wearable device may use the data processed in the watch-type terminal 100 on the wearable device. For example, when a call is received in the watch-type terminal 100, the user may answer the call using the wearable device. Also, when a message is received in the watch-type terminal 100, the user can check the received message using the wearable device.

The location information module 115 is generally configured to detect, calculate, derive or otherwise identify a position of the mobile terminal. As an example, the location information module 115 includes a Global Position System (GPS) module, a Wi-Fi module, or both. If desired, the location information module 115 may alternatively or additionally function with any of the other modules of the wireless communication unit 110 to obtain data related to the position of the mobile terminal. As one example, when the mobile terminal uses a GPS module, a position of the mobile terminal may be acquired using a signal sent from a GPS satellite. As another example, when the mobile terminal uses the Wi-Fi module, a position of the mobile terminal can be acquired based on information related to a wireless access point (AP) which transmits or receives a wireless signal to or from the Wi-Fi module.

The input unit 120 may be configured to permit various types of input to the mobile terminal 120. Examples of such input include audio, image, video, data, and user input. Image and video input is often obtained using one or more cameras 121. Such cameras 121 may process image frames of still pictures or video obtained by image sensors in a video or image capture mode. The processed image frames can be displayed on the display unit 151 or stored in memory 170. In some cases, the cameras 121 may be arranged in a matrix configuration to permit a plurality of images having various angles or focal points to be input to the watch-type terminal 100. As another example, the cameras 121 may be located in a stereoscopic arrangement to acquire left and right images for implementing a stereoscopic image.

The microphone 122 is generally implemented to permit audio input to the watch-type terminal 100. The audio input can be processed in various manners according to a function being executed in the watch-type terminal 100. If desired, the microphone 122 may include assorted noise removing algorithms to remove unwanted noise generated in the course of receiving the external audio.

The user input unit 123 is a component that permits input by a user. Such user input may enable the control unit 180 to control operation of the watch-type terminal 100. The user input unit 123 may include one or more of a mechanical input element (for example, a key, a button located on a front and/or rear surface or a side surface of the watch-type terminal 100, a dome switch, a jog wheel, a jog switch, and the like), or a touch-sensitive input, among others. As one example, the touch-sensitive input may be a virtual key or a soft key, which is displayed on a touch screen through software processing, or a touch key which is located on the mobile terminal at a location that is other than the touch screen. On the other hand, the virtual key or the visual key may be displayed on the touch screen in various shapes, for example, graphic, text, icon, video, or a combination thereof.

The sensing unit 140 is generally configured to sense one or more of internal information of the mobile terminal, surrounding environment information of the mobile terminal, user information, or the like. The control unit 180 generally cooperates with the sending unit 140 to control operation of the watch-type terminal 100 or execute data processing, a function or an operation associated with an application program installed in the mobile terminal based on the sensing provided by the sensing unit 140. The sensing unit 140 may be implemented using any of a variety of sensors, some of which will now be described in more detail.

The proximity sensor 141 may include a sensor to sense presence or absence of an object approaching a surface, or an object located near a surface, by using an electromagnetic field, infrared rays, or the like without a mechanical contact. The proximity sensor 141 may be arranged at an inner region of the mobile terminal covered by the touch screen, or near the touch screen.

The proximity sensor 141, for example, may include any of a transmissive type photoelectric sensor, a direct reflective type photoelectric sensor, a mirror reflective type photoelectric sensor, a high-frequency oscillation proximity sensor, a capacitance type proximity sensor, a magnetic type proximity sensor, an infrared rays proximity sensor, and the like. When the touch screen is implemented as a capacitance type, the proximity sensor 141 can sense proximity of a pointer relative to the touch screen by changes of an electromagnetic field, which is responsive to an approach of an object with conductivity. In this case, the touch screen (touch sensor) may also be categorized as a proximity sensor.

The term "proximity touch" will often be referred to herein to denote the scenario in which a pointer is positioned to be proximate to the touch screen without contacting the touch screen. The term "contact touch" will often be referred to herein to denote the scenario in which a pointer makes physical contact with the touch screen. For the position corresponding to the proximity touch of the pointer relative to the touch screen, such position will correspond to a position where the pointer is perpendicular to the touch screen. The proximity sensor 141 may sense proximity touch, and proximity touch patterns (for example, distance, direction, speed, time, position, moving status, and the like). In general, control unit 180 processes data corresponding to proximity touches and proximity touch patterns sensed by the proximity sensor 141, and cause output of visual information on the touch screen. In addition, the control unit 180 can control the watch-type terminal 100 to execute different operations or process different data according to whether a touch with respect to a point on the touch screen is either a proximity touch or a contact touch.

A touch sensor can sense a touch applied to the touch screen, such as display unit 151, using any of a variety of touch methods. Examples of such touch methods include a resistive type, a capacitive type, an infrared type, and a magnetic field type, among others.

As one example, the touch sensor may be configured to convert changes of pressure applied to a specific part of the display unit 151, or convert capacitance occurring at a specific part of the display unit 151, into electric input signals. The touch sensor may also be configured to sense not only a touched position and a touched area, but also touch pressure and/or touch capacitance. A touch object is generally used to apply a touch input to the touch sensor. Examples of typical touch objects include a finger, a touch pen, a stylus pen, a pointer, or the like.

When a touch input is sensed by a touch sensor, corresponding signals may be transmitted to a touch controller. The touch controller may process the received signals, and then transmit corresponding data to the control unit 180. Accordingly, the control unit 180 may sense which region of the display unit 151 has been touched. Here, the touch controller may be a component separate from the control unit 180, the control unit 180, and combinations thereof.

In some embodiments, the control unit 180 may execute the same or different controls according to a type of touch object that touches the touch screen or a touch key provided in addition to the touch screen. Whether to execute the same or different control according to the object which provides a touch input may be decided based on a current operating state of the watch-type terminal 100 or a currently executed application program, for example.

The touch sensor and the proximity sensor may be implemented individually, or in combination, to sense various types of touches. Such touches includes a short (or tap) touch, a long touch, a multi-touch, a drag touch, a flick touch, a pinch-in touch, a pinch-out touch, a swipe touch, a hovering touch, and the like.

If desired, an ultrasonic sensor may be implemented to recognize position information relating to a touch object using ultrasonic waves. The control unit 180, for example, may calculate a position of a wave generation source based on information sensed by an illumination sensor and a plurality of ultrasonic sensors. Since light is much faster than ultrasonic waves, the time for which the light reaches the optical sensor is much shorter than the time for which the ultrasonic wave reaches the ultrasonic sensor. The position of the wave generation source may be calculated using this fact. For instance, the position of the wave generation source may be calculated using the time difference from the time that the ultrasonic wave reaches the sensor based on the light as a reference signal.

The camera 121 typically includes at least one a camera sensor (CCD, CMOS etc.), a photo sensor (or image sensors), and a laser sensor.

Implementing the camera 121 with a laser sensor may allow detection of a touch of a physical object with respect to a 3D stereoscopic image. The photo sensor may be laminated on, or overlapped with, the display device. The photo sensor may be configured to scan movement of the physical object in proximity to the touch screen. In more detail, the photo sensor may include photo diodes and transistors at rows and columns to scan content received at the photo sensor using an electrical signal which changes according to the quantity of applied light. Namely, the photo sensor may calculate the coordinates of the physical object according to variation of light to thus obtain position information of the physical object.

The display unit 151 is generally configured to output information processed in the watch-type terminal 100. For example, the display unit 151 may display execution screen information of an application program executing at the watch-type terminal 100 or user interface (UI) and graphic user interface (GUI) information in response to the execution screen information.

In some embodiments, the display unit 151 may be implemented as a stereoscopic display unit for displaying stereoscopic images.

A typical stereoscopic display unit may employ a stereoscopic display scheme such as a stereoscopic scheme (a glass scheme), an auto-stereoscopic scheme (glassless scheme), a projection scheme (holographic scheme), or the like.

The audio output module 152 is generally configured to output audio data. Such audio data may be obtained from any of a number of different sources, such that the audio data may be received from the wireless communication unit 110 or may have been stored in the memory 170. The audio data may be output during modes such as a signal reception mode, a call mode, a record mode, a voice recognition mode, a broadcast reception mode, and the like. The audio output module 152 can provide audible output related to a particular function (e.g., a call signal reception sound, a message reception sound, etc.) performed by the watch-type terminal 100. The audio output module 152 may also be implemented as a receiver, a speaker, a buzzer, or the like.

A haptic module 153 can be configured to generate various tactile effects that a user feels, perceive, or otherwise experience. A typical example of a tactile effect generated by the haptic module 153 is vibration. The strength, pattern and the like of the vibration generated by the haptic module 153 can be controlled by user selection or setting by the control unit. For example, the haptic module 153 may output different vibrations in a combining manner or a sequential manner.

Besides vibration, the haptic module 153 can generate various other tactile effects, including an effect by stimulation such as a pin arrangement vertically moving to contact skin, a spray force or suction force of air through a jet orifice or a suction opening, a touch to the skin, a contact of an electrode, electrostatic force, an effect by reproducing the sense of cold and warmth using an element that can absorb or generate heat, and the like.

The haptic module 153 can also be implemented to allow the user to feel a tactile effect through a muscle sensation such as the user's fingers or arm, as well as transferring the tactile effect through direct contact. Two or more haptic modules 153 may be provided according to the particular configuration of the watch-type terminal 100.

An optical output module 154 can output a signal for indicating an event generation using light of a light source. Examples of events generated in the watch-type terminal 100 may include message reception, call signal reception, a missed call, an alarm, a schedule notice, an email reception, information reception through an application, and the like.

A signal output by the optical output module 154 may be implemented in such a manner that the mobile terminal emits monochromatic light or light with a plurality of colors. The signal output may be terminated as the mobile terminal senses that a user has checked the generated event, for example.

The interface unit 160 serves as an interface for external devices to be connected with the watch-type terminal 100. For example, the interface unit 160 can receive data transmitted from an external device, receive power to transfer to elements and components within the watch-type terminal 100, or transmit internal data of the watch-type terminal 100 to such external device. The interface unit 160 may include wired or wireless headset ports, external power supply ports, wired or wireless data ports, memory card ports, ports for connecting a device having an identification module, audio input/output (I/O) ports, video I/O ports, earphone ports, or the like.

The identification module may be a chip that stores various information for authenticating authority of using the watch-type terminal 100 and may include a user identity module (UIM), a subscriber identity module (SIM), a universal subscriber identity module (USIM), and the like. In addition, the device having the identification module (also referred to herein as an "identifying device") may take the form of a smart card. Accordingly, the identifying device can be connected with the terminal 100 via the interface unit 160.

When the watch-type terminal 100 is connected with an external cradle, the interface unit 160 can serve as a passage to allow power from the cradle to be supplied to the watch-type terminal 100 or may serve as a passage to allow various command signals input by the user from the cradle to be transferred to the mobile terminal there through. Various command signals or power input from the cradle may operate as signals for recognizing that the mobile terminal is properly mounted on the cradle.

The memory 170 can store programs to support operations of the control unit 180 and store input/output data (for example, phonebook, messages, still images, videos, etc.). The memory 170 may store data related to various patterns of vibrations and audio which are output in response to touch inputs on the touch screen.

The memory 170 may include one or more types of storage mediums including a Flash memory, a hard disk, a solid state disk, a silicon disk, a multimedia card micro type, a card-type memory (e.g., SD or DX memory, etc), a Random Access Memory (RAM), a Static Random Access Memory (SRAM), a Read-Only Memory (ROM), an Electrically Erasable Programmable Read-Only Memory (EEPROM), a Programmable Read-Only memory (PROM), a magnetic memory, a magnetic disk, an optical disk, and the like. The watch-type terminal 100 may also be operated in relation to a network storage device that performs the storage function of the memory 170 over a network, such as the Internet.

The control unit 180 may typically control the general operations of the watch-type terminal 100. For example, the control unit 180 may set or release a lock state for restricting a user from inputting a control command with respect to applications when a status of the mobile terminal meets a preset condition.

The control unit 180 can also perform the controlling and processing associated with voice calls, data communications, video calls, and the like, or perform pattern recognition processing to recognize a handwriting input or a picture drawing input performed on the touch screen as characters or images, respectively. In addition, the control unit 180 can control one or a combination of those components in order to implement various exemplary embodiments disclosed herein.

The power supply unit 190 receives external power or provide internal power and supply the appropriate power required for operating respective elements and components included in the watch-type terminal 100. The power supply unit 190 may include a battery, which is typically rechargeable or be detachably coupled to the terminal body for charging.

The power supply unit 190 may include a connection port. The connection port may be configured as one example of the interface unit 160 to which an external charger for supplying power to recharge the battery is electrically connected.

As another example, the power supply unit 190 may be configured to recharge the battery in a wireless manner without use of the connection port. In this example, the power supply unit 190 can receive power, transferred from an external wireless power transmitter, using at least one of an inductive coupling method which is based on magnetic induction or a magnetic resonance coupling method which is based on electromagnetic resonance.

Various embodiments described herein may be implemented in a computer-readable medium, a machine-readable medium, or similar medium using, for example, software, hardware, or any combination thereof.

FIG. 2 is a perspective view illustrating a watch-type terminal viewed in one direction according to an embodiment.

Referring to FIG. 2, the watch-type terminal 100 includes a main body 101 with a display unit 151 and a band 300 connected to the main body 101 to be wearable on a wrist.

The main body 101 may include a case having a certain appearance. As illustrated, the case may include a first case 101a and a second case 101b cooperatively defining an inner space for accommodating various electronic components. Other configurations are possible. For instance, a single case may alternatively be implemented, with such a case being configured to define the inner space, thereby implementing a mobile terminal 100 with a uni-body.

The watch-type terminal 100 can perform wireless communication, and an antenna for the wireless communication can be installed in the main body 101. The antenna may extend its function using the case. For example, a case including a conductive material may be electrically connected to the antenna to extend a ground area or a radiation area.

The display unit 151 is shown located at the front side of the main body 101 so that displayed information is viewable to a user. In some embodiments, the display unit 151 includes a touch sensor so that the display unit can function as a touch screen. As illustrated, a window 151a is positioned on the first case 101a to form a front surface of the terminal body together with the first case 101a.

The illustrated embodiment includes audio output module 152, a camera 121, a microphone 122, and a user input unit 123 positioned on the main body 101. When the display unit 151 is implemented as a touch screen, additional function keys may be minimized or eliminated. For example, when the touch screen is implemented, the user input unit 123 may be omitted.

The band 300 is commonly worn on the user's wrist and may be made of a flexible material for facilitating wearing of the device. As one example, the band 300 may be made of fur, rubber, silicon, synthetic resin, or the like. The band 300 may also be configured to be detachable from the main body 101. Accordingly, the band 300 may be replaceable with various types of bands according to a user's preference.

In one configuration, the band 300 may be used for extending the performance of the antenna. For example, the band may include therein a ground extending portion (not shown) electrically connected to the antenna to extend a ground area.

The band 300 may include a fastener 330. The fastener 330 may be implemented into a buckle type, a snap-fit hook structure, a Velcro® type, or the like, and include a flexible section or material. The drawing illustrates an example that the fastener 330 is implemented using a buckle.

A watch-type terminal according to the present embodiment includes an electrode unit 340 supplying a current to a user's body to collect biometric information of the user. The electrode unit 340 supplies a current to the user's body and the watch-type terminal forms an impedance value using voltage values at specific two points. A result of body fat measurement result of the user may be provided using the impedance value.

The watch-type terminal 100 according to an embodiment of the present invention includes a second band 320 for collecting biometric information and a first band 310 for fixing to the user's wrist. The second band 320 includes the electrode unit 340. The second band 320 is electrically connected to the main body 101.

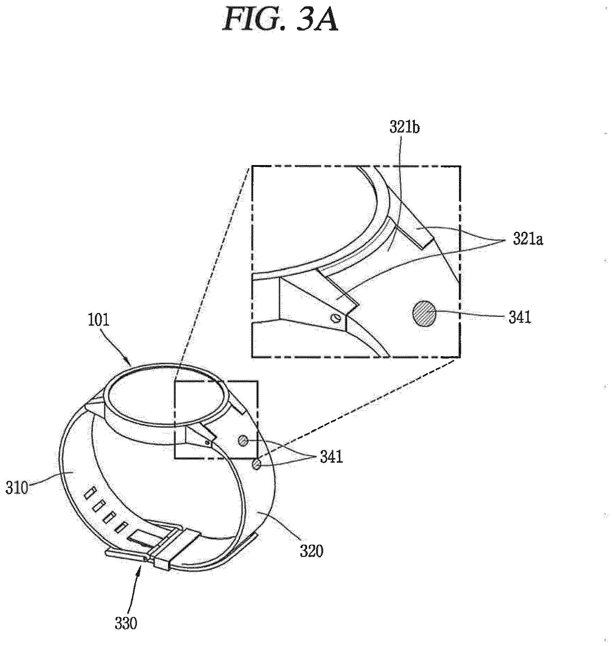

FIGS. 3A to 3C are conceptual diagrams illustrating a structure in which first and second bands are fixed to the main body.

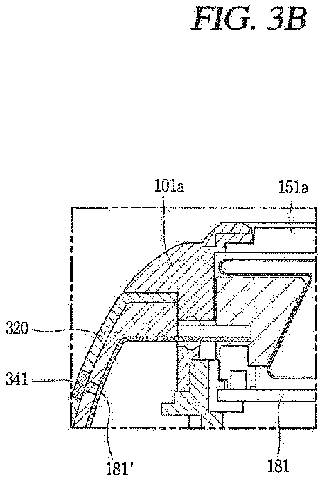

Referring to FIGS. 2, 3A, and 3B, the electrode unit 340 includes first and second electrode members 341 and 342. The first electrode member 341 is formed to be exposed on a first surface of the second band 320 and the second electrode member 342 is exposed on a second surface of the second band 320. The first and second surfaces correspond to opposite surfaces.

The first surface on which the first electrode member 341 is formed corresponds to a surface of the watch-type terminal 100 which comes into contact with the user's wrist when the watch-type terminal 100 is worn on the user's wrist. The first electrode member 341 may protrude from a surface of the first surface so that the first surface may contact the user's wrist.

The second electrode member 342 is formed on the second surface exposed to the outside when the watch-type terminal 100 is worn on the user's wrist. The first and second electrode members 341 and 342 may be disposed to overlap each other, but the present invention is not limited thereto.

The first and second electrode members 341 and 342 are made of metal members capable of supplying a current and may be formed of a pair of metal members as shown in the drawing. For example, the first electrode member 341 may be formed of a pair of metal members spaced apart from each other, and the second electrode member 342 may be formed of metal members spaced apart from each other with the first electrode member 341 interposed therebetween. However, a disposition structure of the metal members is not limited thereto.

One of the first electrode member 341 or the second electrode member 342 may be configured as a Tx electrode supplying current and the other electrode member may be configured as an Rx electrode. The electrode member configured as the Tx electrode supplies a current into the body continuously or on the basis of a specific control command.

When the user's body comes into contact with the first and second electrode members 341 and 342 to form a closed loop in which a current flows, a voltage is formed by the current flowing through the body. The controller 180 measures the voltage.

For example, when the watch-type terminal 100 is detected to be worn on the user's wrist, the controller may control the electrode unit 340 to supply current or control the electrode unit 340 to supply current at a predetermined time interval,. Or, when it is detected that both the first and second electrode members 341 and 342 are in contact with the body, the controller may control the electrode unit 340 to supply current.

Referring to FIGS. 3A and 3B, the second band 320 may be closely fixed to the main body 101. A protruding part 321b formed at a region of the end of the second band 320 is connected to a second fixing part 321a protruding from the main body 101. The second band 320 includes a flexible circuit board 181' electrically connected to the first and second electrode members 341 and 342. The flexible circuit board 181' is formed inside the second band 320. The flexible circuit board 181' extends from the second band 320 and is inserted into the main body 101. Although not specifically shown in the drawing, the flexible circuit board 181' is electrically connected to both the first and second electrode members 341 and 342.

In FIG. 3A, it is illustrated that the first electrode member 341 is connected to the flexible circuit board 181' by an additional connection member, but the present invention is not limited thereto. For example, the first electrode member 341 may have a predetermined thickness and may be directly connected to the flexible circuit board 181'.

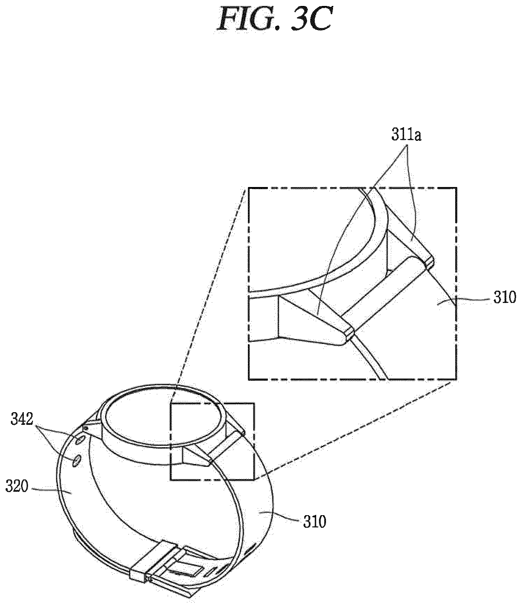

Referring to FIG. 3C, the first band 310 may not include a structure electrically connected to the main body 101. The first band 310 is fixed by a first fixing part 311a protruding from the main body 101. The first fixing part 311a may include a hinge structure to rotatably fix the first band 310.

Alternatively, the first band 310 may include a structure having an antenna (NFC, LTE, GPS) for performing wireless communication and electrically connected to the main body 101.

According to this embodiment, when the watch-type terminal is worn, the first electrode member 341 contacts the user's body, and when the user intentionally touches the second electrode member 342, a voltage based on a current passing through a region of the user's body is measured.

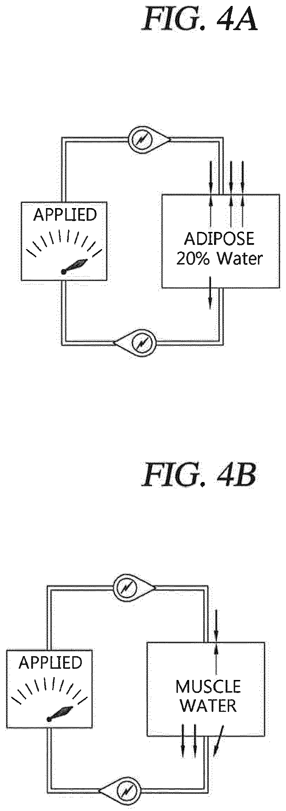

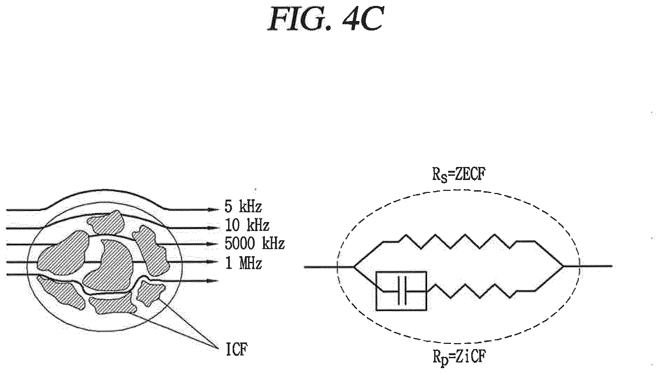

FIGS. 4A to 5B are conceptual diagrams illustrating a body fat measurement principle. Referring to FIGS. 4A and 4B, electrical conductivity of the adipose and electrical conductivity of the muscle are different from each other. Lean mass contains the most body water in the body and has conductor properties when a fine current is supplied thereto, and body fat rarely contains water and thus has nonconductive properties that current does not flow easily. A weight is determined by the sum of body fat and fat free mass, and fat free mass is formed by the sum of soft lean mass and mineral mass.

Therefore, although the same current is supplied, voltage values are measured to be different according to amounts of fat and muscle included in a region of the body. Fat has nonconductive properties in which current does not flow easily and has a high impedance value. Muscle has properties such as a conductor in which current flows easily and has low impedance. That is, the controller 180 may obtain a body fat measurement result using the impedance value.

The components of the user's body may be measured by adjusting a frequency. Membranes of the cells of the body act like a resistive membrane and have characteristics that impedance is lowered only when a frequency is raised. That is, a low frequency passes only outside the tissues of cells, but a high frequency form pathways within and outside the tissues of cells. For example, a current of about 1 KHz may measure conductivity of the skin of the outer layer, and a current of about 50 KHz may measure impedance of the body's components. Therefore, the first and second electrode members 341 and 342 allow a current to reach cells using a high frequency.

That is, regarding the body as a resistor, when a low current of a specific frequency (about 50 KHz) is supplied between the first and second electrode members 341 and 342, a voltage is formed. Accordingly, impedance information on a part of the body in which a current flows between the first and second electrode members 341 and 342 may be obtained using a voltage and a current. For example, the controller 180 may obtain an impedance value regarding a part of the body between the first and second electrode members 341 and 342, and may correct the impedance value on the basis of user information (age, sex, height, weight, etc.) to form a body fat measurement result including an amount of muscle and an amount of fat.

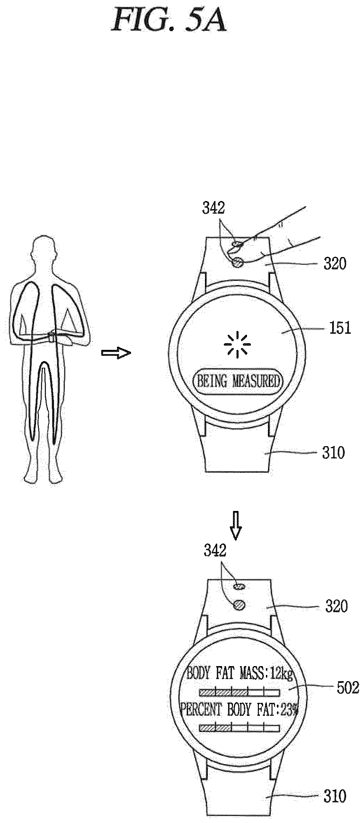

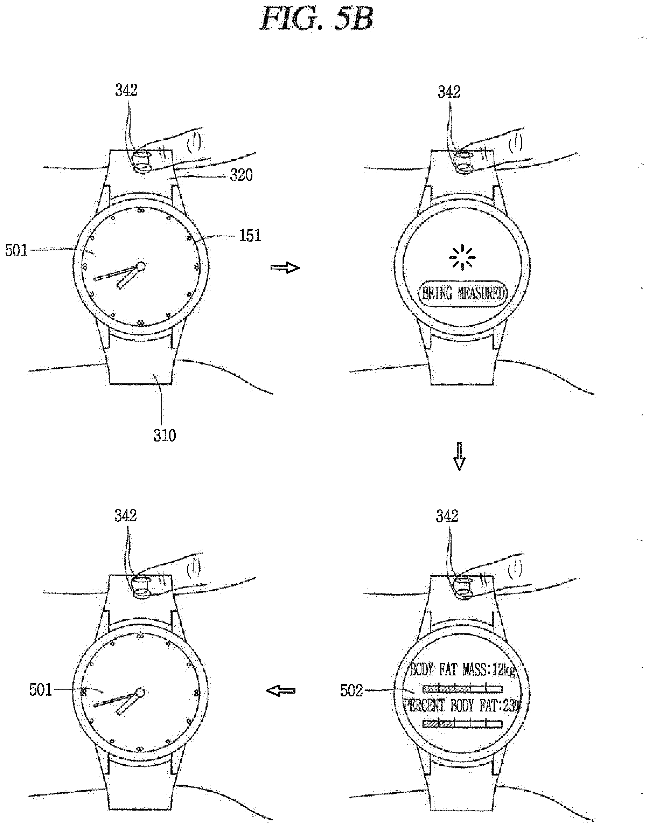

FIGS. 5A and 5B are conceptual views illustrating a control method of a watch-type terminal according to an embodiment of the present invention.

Referring to FIG. 5A, an application for providing a body fat measurement result to the watch-type terminal 100 according to an embodiment of the present invention may be installed. When the application is executed, the controller 180 may perform control to store user information (age, gender, height, weight, etc.) of the user in the memory 170.

When the user brings a part (for example, a finger) of the body into contact with the second electrode member 342 in a state where the first electrode member 341 is in contact with the user's wrist, the controller applies a current to measure a voltage. As shown in the drawing, a current passes through one area of the user's body by the first and second electrode members 341 and 342 in contact with one wrist and one finger, respectively. For example, in case where the watch-type terminal 100 is worn on the right wrist and the left hand is brought into contact with the second electrode member 342, a voltage regarding a current flowing from the right arm to the left hand through both legs may be measured. The display unit 151 may output a notification screen indicating a measurement state while a current is output.

The controller 180 calculates a body fat measurement result on the basis of the impedance value and the user information stored in the memory 170. The display unit 151 outputs the body fat measurement result 502. The controller 180 may control the haptic module 153 to output vibration, while the impedance value is being measured.

Referring to FIG. 5B, when a finger is brought into contact with the second electrode member 342 while time information 501 is being output on the display unit 15, the controller 180 outputs a body fat measurement result 502 using a current flowing through the body. After the body fat measurement result 502 is output, when a preset time t has lapsed, the controller controls the display unit 151 to output the time information 501 again.

The display unit 151 may continuously output the time information 501 while the voltage is being measured.

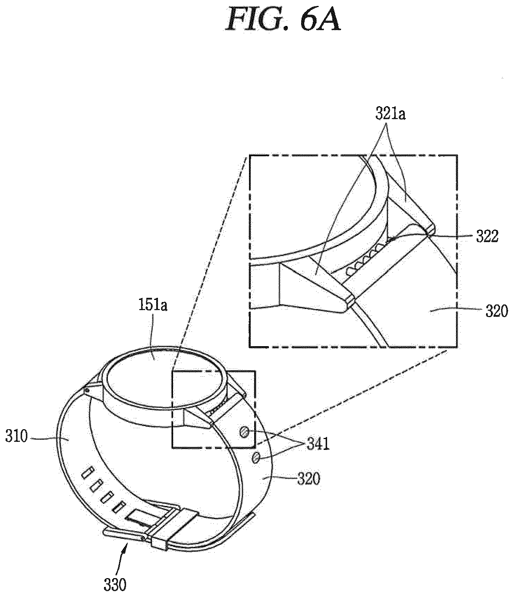

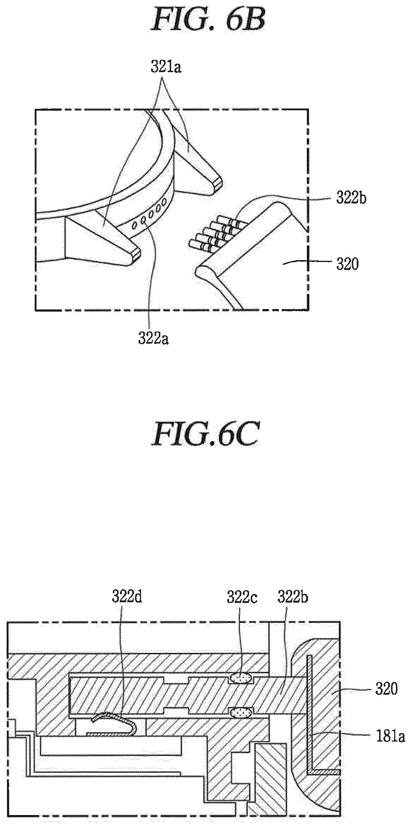

FIG. 6A is a conceptual view illustrating a structure of a watch-type terminal according to another embodiment. FIG. 6B is a partially enlarged view illustrating a connecting part, and FIG. 6C is a sectional view of the connecting part. The other components of the watch-type terminal 100 according to the present embodiment are substantially the same as or similar to those of FIG. 3A except for some components of the second band 320 are substantially the same as or similar to the components of FIG. 3A. Therefore, the same reference numerals are given to the same components and redundant explanations will be omitted.

An electrode unit of the second band 320 according to FIGS. 3A and 6A to 6C includes a first electrode member 341 formed on a first surface and a second electrode member 342 formed on a second surface.

The second band 320 is fixed by a fixing part 321a protruding from the main body 101. The fixing part 321a may include a hinge structure and rotatably connect the second band 320 to the main body 101. Alternatively, the fixing part 321a may be formed to support a portion of both sides of the second band 320. In this case, the second band 320 may be detachably coupled to the main body 101.

A connection part 322 transmitting an electrical signal is formed between the end of the second band 320 and the main body 101. The connection part 322 may include a connection pin 322b protruding from the second band 320 and fitting into one region of the main body 101.

Referring to FIG. 6B, the connection part 322 includes a connection hole 322a formed on the main body 101 and a connection pin 322b inserted into the connection hole 322a. The connection hole 322a is disposed between the fixing parts 321a and may be formed in plurality, and the connection pin 322b may also be formed in plurality. For example, when the first and second electrode members 341 and 342 are each formed of a pair of metal members, four connection pins 322b respectively connected to the four metal members may be formed.

The second band 320 of the watch-type terminal 100 according to the present embodiment is detachable from the main body 101. That is, the connection pin 322b is separable from the connection hole 322a. The second band 320 is fixed to the main body 101 when the connection pin 322b is inserted into the connection hole 322a.

Referring to FIG. 3C, the connection pin 322b is connected to a flexible circuit board 181a installed within the second band 320 and electrically connected to the electrode unit 340. When the connection pin 322b outwardly protruding from the second band 320 is inserted into the connection hole 322a, the connection pin 322b is fixed by a fixing protrusion 322c formed within the connection hole 322a. An outer circumferential surface of the connection pin 322b has a seating groove allowing the fixing protrusion 322c to be seated thereon. The connection pin 322b is prevented from being separated from the connection hole 322a by virtue of the fixing protrusion 322c.

A connection member 322d electrically connected to the circuit board 181 mounted inside the main body 101 is disposed within the connection hole 322a. The connection member 322d may be connected to each of the plurality of connection pins 322b.

Although not shown specifically in the drawing, the main body 101 may be provided with an additional band different from the second band 320. For example, the additional band may include an electronic component that performs a function different from that of the second band 320 may be formed of a strap that does not include any electronic component.

For example, the additional band may be a bio band for collecting biometric information of the body. The bio-band may include at least one of an electrocardiogram (ECG) sensor, a GSR sensor, and a fingerprint sensor.

The electrocardiogram sensor, a sensor for measuring electrical activity of the heart, may recognize a heart condition of the user. The GSR sensor detects an electrical resistance or a change in an action potential by bringing an electrode into contact with the skin, and may analyze the user's feelings, emotions, and the like, using the electrical resistance or the change in the action potential. When such sensors are installed, the user's biometric information may be periodically collected while the user wears the watch-type terminal 100.

In addition, the user may perform various controls by recognizing the finger by the fingerprint sensor. Preferably, the fingerprint sensor is formed on one surface of the watch-type terminal 100 exposed to the outside, while the watch-type terminal 100 is worn on.

Although it is described that the sensors are mounted on the additional band, the sensors may also be formed on the second band 320 together with the electrode unit 340. Or, different sensors may be mounted for the respective bands so that the sensors may be alternately mounted on the main body 101.

According to this embodiment, since the second band formed with the electrode unit for forming the body fat result information of the user may be detachably attached to the main body, the user may use the second band only when desired, and may collect desired biometric information by using the band in which different sensors are installed.

That is, since the electrode units are all formed on the band, the user may receive the body fat information only when desired, and a weight of the main body may be reduced.

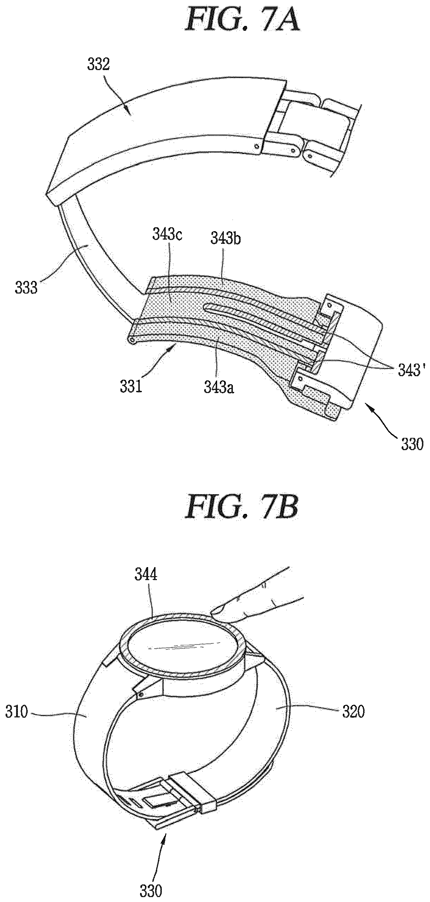

FIGS. 7A to 7D are conceptual views illustrating a watch-type terminal including an electrode unit according to various embodiments.

Referring to FIG. 7A, a fastener 330 according to the present embodiment includes first and second regions 331 and 332 which overlap each other when coupled and a connection region 333 connecting the first and second regions 331 and 332. The first and second regions 331 and 332 may further include fitting structures fixed to each other when coupled.

A portion of the electrode units 340 according to the present embodiment may be formed as a component of the first region 331. The first region 331 is formed to contact the wrist of the user when the second region 332 is coupled. The first region 331 is made of a metal material. In the electrode unit 340, a first electrode member 343 may be formed in the first region 331. The first electrode member 343 may be formed as a Rx or Tx electrode.

The first electrode member 343 may include first and second metal members 343a and 343b, a support part 343c supporting the first and second metal members 343a and 343b, and an insulating part 343' preventing electrical connection between the first and second metal members 343a and 343b. The first and second electrode members 343a and 343b may extend in one direction and may be disposed in parallel with each other.

Although not shown in the drawing, the first and second electrode members 343a and 343b are installed within the second band 320 and are electrically connected to the flexible circuit board connected to the main body 101.

The watch-type terminal 100 according to the present embodiment includes a second electrode member formed in a region of the band or the main body 101.

Referring to FIG. 7B, a second electrode member 344 may be formed on the main body 101. The second electrode member 344 may be formed in a region surrounding the edge of the display unit 151. The second electrode member 344 is formed to be exposed to the outside when the watch-type terminal 100 is worn on the user's wrist.

A shape of the second electrode member 344 is not limited to that shown in FIG. 7B. For example, the second electrode member 344 may include a plurality of metal members disposed in a region of the first case 101a surrounding the display unit 151 and spaced apart from each other. When the second electrode member 344 includes the plurality of metal members, an insulator may be provided between the plurality of metal members.

Referring to FIGS. 7A and 7B, when the watch-type terminal 100 is worn on the wrist, the first electrode member 343 contacts the user's body and the second electrode member 344 is exposed to the outside. Accordingly, when the user brings a part of his body such as the hand, or the like, into contact with the second electrode member 344, a current flows through the part of the user's body and a corresponding impedance value may be calculated.

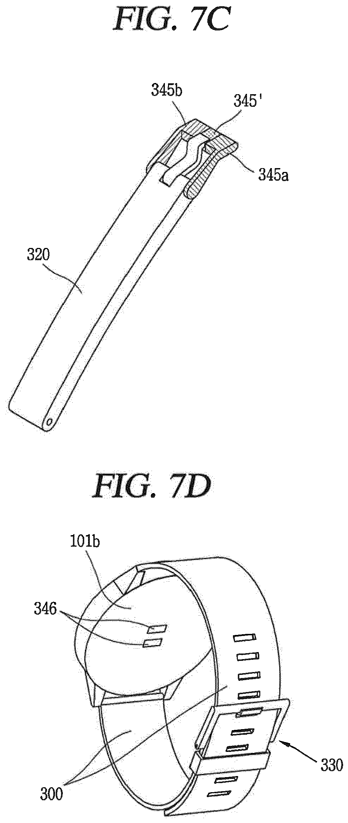

FIG. 7C illustrates a structure of the first electrode member 343 according to another embodiment. The first electrode member 343 according to this embodiment is formed on the fastener connecting the first and second bands. The first electrode member 343 includes a first metal member 345a and a second metal member 345b which are connected to the second band 320. The first and second metal members 345a and 345b are connected to an insulating part 345'. The first and second metal members 345a and 345b may be formed as an Rx or Tx electrode. One end of the first band may be inserted into a space formed between the first and second metal members 345a and 345b and the second band 320.

The watch-type terminal 100 according to the present embodiment includes the first electrode member 343 formed in one region of the main body or the band and the second electrode member forming a closed loop through which current flows.

Referring to FIG. 7D, a first electrode member 346 is formed on the second case 101b of the main body 101. The first electrode member 346 may include a plurality of metal members mounted on an outer surface of the second case 101b, but the present invention is not limited thereto. The second case 101b itself may be made of a metal case. The first electrode member 346 according to the present embodiment comes into contact with the user's body when the watch-type terminal 100 is worn, and the second electrode member forming a closed loop in which a current flows with the first electrode member 343 is preferably formed in one region exposed to the outside when the watch-type terminal 100 is worn.

Also, although not shown in the drawings, the second electrode member may be formed in a region of an external device which is available to be wirelessly connected with the watch-type terminal. In this case, when the watch-type terminal 100 is worn, a current applied from the first electrode member is transmitted to an electrode member of the external device through one region of the human body.

In this case, user information (age, sex, height, weight, etc.) for forming a body fat measurement result may be received from the external device or the calculated body fat measurement result may be controlled to be transmitted to the wirelessly connected external device.

The external device may be a mobile phone, a smartphone, a laptop computer, a digital broadcasting terminal, a personal digital assistant (PDA), a portable multimedia player (PMP), a navigation device, a slate PC, a tablet PC, an ultrabook, a wearable device (e.g., a smartwatch, a smart glass, or a head mounted display (HMD)), and the like.

The present invention described above may be implemented as a computer-readable code in a medium in which a program is recorded. The computer-readable medium includes any type of recording device in which data that may be read by a computer system is stored. The computer-readable medium may be, for example, a hard disk drive (HDD), a solid state disk (SSD), a silicon disk drive (SDD), a ROM, a RAM, a CD-ROM, a magnetic tape, a floppy disk, an optical data storage device, and the like. The computer-readable medium also includes implementations in the form of carrier waves (e.g., transmission via the Internet). Also, the computer may include the controller 180 of the terminal. Thus, the foregoing detailed description should not be interpreted limitedly in every aspect and should be considered to be illustrative. The scope of the present invention should be determined by reasonable interpretations of the attached claims.

### [Industrial Applicability]

The present embodiments relate to a watch-type terminal for promptly providing a body fat measurement result by bringing a part of a human body into contact with the watch-type terminal, and thus, the present embodiments may be applied to various related industrial fields

## Claims

1. A watch-type terminal wearable on a user's wrist, the watch-type terminal comprising:
a main body (101);
a band part connected to the main body (101) and formed to be wearable on the wrist;
an electrode unit (340) disposed in the band so as to come into contact with the body of the user, and generating a current; and
a controller (180) configured to calculate an impedance value on the basis of a voltage sensed by the electrode unit (340),
wherein the electrode unit (340) includes a first electrode member (341, 343, 346) which comes into contact with the wrist when the watch-type terminal is worn around the wrist and a second electrode member (342, 344) exposed to the outside,
wherein the band part includes a first band (310) and a second band (320) connected to both ends of the main body (101),
wherein the electrode unit (340) includes the first electrode member (341) formed on one surface of the second band (320) and the second electrode member (342) formed on the other surface of the second band (320),
wherein the first band (310) is fixed to the main body (101), and the second band (320) is detachable from the main body, and
wherein the second band (320) further includes:
a flexible circuit board electrically connecting the first and second electrode members (341, 342), and
a connection pin (322b) connected to the flexible circuit board and protruding to the outside of the second band (320), and
wherein the main body (101) further includes a connection hole (322a) allowing the connection pin (322b) to be inserted therein.

2. The watch-type terminal of claim 1, wherein when a region of the human body is brought into contact with the second electrode member (342), the controller (180) is configured to calculate the impedance value using a voltage sensed between the first and second electrode members (341, 342).

3. The watch-type terminal of claim 1, wherein
when the first and second electrode members (341, 342) are formed as a plurality of metal members, the second band (320) includes a plurality of connection pins respectively connected to the plurality of metal members.

4. The watch-type terminal of claim 1, further comprising:
at least one sensor formed on one surface of the second band (320) and sensing a biometric signal.

5. The watch-type terminal of claim 1, wherein
the main body (101) includes:
a display unit (151) forming one surface and outputting screen information;
a first case (101a) surrounding the display unit; and
a second case (101b) coupled to the first case (101a) and facing the first case (101a).

6. The watch-type terminal of claim 1, wherein
the controller (180) is configured to calculate a body fat measurement result on the basis of the impedance value and user information stored in the memory.

7. The watch-type terminal of claim 1, wherein
the controller (180) is configured to control a haptic module (153) to output vibration, while the impedance value is being measured.

8. The watch-type terminal of claim 1, wherein when a user's finger comes into contact with the second electrode member (342, 344), the controller (180) is configured to output a body fat measurement result using a current value flowing through the body of the user.

## Patentansprüche

1. Uhr-Endgerät, das am Handgelenk eines Benutzers getragen werden kann, wobei das Uhr-Endgerät umfasst:
einen Hauptkörper (101);
einen Bandteil, der mit dem Hauptkörper (101) verbunden und so geformt ist, dass er am Handgelenk getragen werden kann;
eine Elektrodeneinheit (340), die in dem Band so angeordnet ist, dass sie mit dem Körper des Benutzers in Kontakt kommt und einen elektrischen Strom erzeugt; und
eine Steuerung (180), die dazu eingerichtet ist, einen Impedanzwert auf der Grundlage einer von der Elektrodeneinheit (340) erfassten Spannung zu berechnen,
wobei die Elektrodeneinheit (340) ein erstes Elektrodenelement (341, 343, 346), das mit dem Handgelenk in Kontakt kommt, wenn das Uhr-Endgerät um das Handgelenk getragen wird, und ein zweites Elektrodenelement (342, 344), das zur Außenseite hin freiliegt, umfasst,
wobei der Bandteil ein erstes Band (310) und ein zweites Band (320) umfasst, die mit beiden Enden des Hauptkörpers (101) verbunden sind,
wobei die Elektrodeneinheit (340) das erste Elektrodenelement (341), das auf einer Oberfläche des zweiten Bandes (320) ausgebildet ist, und das zweite Elektrodenelement (342), das auf der anderen Oberfläche des zweiten Bandes (320) ausgebildet ist, enthält,
wobei das erste Band (310) an dem Hauptkörper (101) befestigt ist und das zweite Band (320) von dem Hauptkörper abnehmbar ist, und
wobei das zweite Band (320) ferner umfasst:
eine flexible Leiterplatte, die das erste und das zweite Elektrodenelement (341, 342) elektrisch verbindet, und
einen Verbindungsstift (322b), der mit der flexiblen Leiterplatte verbunden ist und aus dem zweiten Band (320) nach außen vorsteht, und
wobei der Hauptkörper (101) ferner ein Verbindungsloch (322a) aufweist, in das der Verbindungsstift (322b) eingeführt werden kann.

2. Uhr-Endgerät gemäß Anspruch 1, wobei
wenn ein Bereich des menschlichen Körpers mit dem zweiten Elektrodenelement (342) in Kontakt gebracht wird, die Steuerung (180) dazu eingerichtet ist, den Impedanzwert unter Verwendung einer zwischen dem ersten und dem zweiten Elektrodenelement (341, 342) gemessenen Spannung zu berechnen.

3. Uhr-Endgerät gemäß Anspruch 1, wobei
wenn die ersten und zweiten Elektrodenelemente (341, 342) als eine Vielzahl von Metallelementen ausgebildet sind, das zweite Band (320) eine Vielzahl von Verbindungsstiften enthält, die jeweils mit der Vielzahl von Metallelementen verbunden sind.

4. Uhr-Endgerät gemäß Anspruch 1, ferner umfassend:
mindestens ein Sensor, der auf einer Oberfläche des zweiten Bandes (320) ausgebildet ist und ein biometrisches Signal erfasst.

5. Uhr-Endgerät gemäß Anspruch 1, wobei
der Hauptteil (101) umfasst:
eine Anzeigeeinheit (151), die eine Fläche bildet und Bildschirminformationen ausgibt;
ein erstes Gehäuse (101a), das die Anzeigeeinheit umgibt; und
ein zweites Gehäuse (101b), das an das erste Gehäuse (101a) gekoppelt ist und dem ersten Gehäuse (101a) zugewandt ist.

6. Uhr-Endgerät gemäß Anspruch 1, wobei
die Steuerung (180) dazu eingerichtet ist, ein Körperfett-Messergebnis auf der Grundlage des Impedanzwertes und im Speicher gespeicherten Benutzerinformationen zu berechnen.

7. Uhr-Endgerät gemäß Anspruch 1, wobei
die Steuerung (180) dazu eingerichtet ist, ein Haptikmodul (153) zu steuern, um Schwingungen auszugeben, während der Impedanzwert gemessen wird.

8. Uhr-Endgerät gemäß Anspruch 1, wobei
wenn ein Finger eines Benutzers mit dem zweiten Elektrodenelement (342, 344) in Berührung kommt, die Steuerung (180) dazu eingerichtet ist, ein Körperfett-Messergebnis unter Verwendung eines durch den Körper des Benutzers fließenden Stromwerts auszugeben.

## Revendications

1. Terminal de type montre pouvant être porté au poignet d'un utilisateur, le terminal de type montre comprenant :
un corps principal (101) ;
une parie de bracelet relié au corps principal (101) et formé pour être porté autour du poignet ;
une unité d'électrodes (340) disposée dans le bracelet de manière à entrer en contact avec le corps de l'utilisateur, et produire du courant ; et
un organe de commande (180) conçu pour calculer une valeur d'impédance sur la base d'une tension détectée par l'unité d'électrodes (340),
l'unité d'électrodes (340) comprenant un premier élément d'électrode (341, 343, 346) qui vient en contact avec le poignet lorsque le terminal de type montre est placé autour du poignet et un second élément d'électrode (342, 344) visible de l'extérieur,
la partie de bracelet comprenant un premier bracelet (310) et un second bracelet (320) relié aux deux extrémités du corps principal (101),
l'unité d'électrodes (340) comprenant le premier élément d'électrode (341) formé sur une face du second bracelet (320) et le second élément d'électrode (342) formé sur l'autre face du second bracelet (320),
le premier bracelet (310) étant fixé au corps principal (101), et le second bracelet (320) pouvant se détacher du corps principal, et
le second bracelet (320) comprenant en outre :
une carte de circuit imprimé souple connectée électroniquement aux premier et second éléments d'électrode (341,342), et
une broche de connexion (322b) connectée à la carte de circuit imprimé souple et faisant saillie vers l'extérieur du second bracelet (320), et
le corps principal (101) comprenant en outre un trou de connexion (322a) où vient s'insérer la broche de connexion (322b).

2. Terminal de type montre selon la revendication 1, lorsqu'une zone du corps humain est mise en contact avec le second élément d'électrode (342), l'organe de commande (180) est conçu pour calculer la valeur d'impédance à l'aide d'une tension détectée entre les premier et second éléments d'électrode (341, 342).

3. Terminal de type montre selon la revendication 1, lorsque les premier et second éléments d'électrode (341, 342) se présentent sous la forme d'une pluralité d'éléments métalliques, le second bracelet (320) comprenant une pluralité de broches de connexion connectées respectivement à la pluralité d'éléments métalliques.

4. Terminal de type montre selon la revendication 1, comprenant en outre :
au moins un capteur formé sur une face du second bracelet (320) et détectant un signal biométrique.

5. Terminal de type montre selon la revendication 1,
le corps principal (101) comprenant :
une unité d'affichage (151) formant une face et affichant des informations d'écran ;
un premier boîtier (101a) entourant l'unité d'affichage ; et
un second boîtier (101b) couplé au premier boîtier (101a) et faisant face au premier boîtier (101a).

6. Terminal de type montre selon la revendication 1,
l'organe de commande (180) étant conçu pour calculer un résultat de mesure des réserves lipidiques de l'organisme en fonction de la valeur d'impédance et des informations utilisateurs mises en mémoire dans la mémoire.

7. Terminal de type montre selon la revendication 1,
l'organe de commande (180) étant conçu pour commander un module haptique (153) pour fournir des vibrations au moment où la valeur d'impédance est mesurée.

8. Terminal de type montre selon la revendication 1,
lorsque le doigt de l'utilisateur entre en contact avec le second élément d'électrode (342, 344), l'organe de commande (180) étant conçu pour délivrer un résultat de mesure des réserves lipidiques de l'organisme à l'aide d'une valeur actuelle circulant dans le corps de l'utilisateur.
